Europäisches Patentamt

European Patent Office

Office européen des brevets

(19)

(11) Veröffentlichungsnummer: **0 062 225**
**B1**

(12) **EUROPÄISCHE PATENTSCHRIFT**

(45) Veröffentlichungstag der Patentschrift:
19.06.85

(51) Int. Cl.⁴: **A 61 K 9/14**, A 61 K 9/20

(21) Anmeldenummer: **82102379.3**

(22) Anmeldetag: **23.03.82**

---

(54) **Sprühgetrocknetes Vitamin-E-Pulver.**

---

(30) Priorität: **06.04.81 US 251076**

(43) Veröffentlichungstag der Anmeldung:
**13.10.82 Patentblatt 82/41**

(45) Bekanntmachung des Hinweises auf die Patenterteilung:
**19.06.85 Patentblatt 85/25**

(84) Benannte Vertragsstaaten:
**CH DE FR GB LI**

(56) Entgegenhaltungen:
**EP - A - 0 005 529**
**US - A - 3 124 510**
**US - A - 3 608 083**

**CHEMICAL ABSTRACTS, Band 89, Nr. 24, Dezember 1978, Seiten 417,418, Nr. 204228c, Columbus, Ohio, USA**

**Die Akte enthält technische Angaben, die nach dem Eingang der Anmeldung eingereicht wurden und die nicht in dieser Patentschrift enthalten sind.**

(73) Patentinhaber: **BASF WYANDOTTE CORPORATION,**
**1609 Biddle Avenue, Wyandotte Michigan 48192 (US)**

(72) Erfinder: **Schmidt, Douglas Norbert, 27780 Johnson, Grosse Ile Michigan 48138 (US)**
Erfinder: **Fischetti, Frank Jr., 42-21 172nd Street, Flushing New York 11358 (US)**

(74) Vertreter: **von Günner, Kurt, Dr. et al, c/o BASF Aktiengesellschaft Carl-Bosch-Strasse 38, D-6700 Ludwigshafen (DE)**

---

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**0 062 225**

### Beschreibung

Die vorliegende Erfindung betrifft ein sprühgetrocknetes Vitamin-E-Pulver, das direkt tablettiert werden kann und Vitamin-E-Acetat, Kaseinat, hydrolysierte Gelatine und Siliziumdioxid enthält.

Die Herstellung von Vitamin-E-Pulvern mit einem Gehalt von 40 bis 60 Gew.-% hydrolysierte Gelatine wird im US-Patent 3 608 083 (Bunnell et al, 21. 09. 1971) beschrieben. Die europäische Patentschrift 5 529 betrifft ein Verfahren zur Herstellung von Vitamin-E-Pulver aus einer Emulsion, die Natrium- oder Kaliumkaseinat enthält, das in einer aus der Laktoseherstellung stammenden Restflüssigkeit gelöst ist. US-Patent 3 962 384 beschreibt ein Verfahren zur Sprühtrocknung von Vitaminpulvern mit Siliziumdioxid.

Probleme, die bei bereits bekannten Pulvern, wie sie z. B. in den oben genannten Literaturstellen beschrieben werden, auftreten, sind z. B. das Zusammenbacken der Teilchen, unbefriedigende Farbe und/oder unbefriedigender Geruch sowie mangelnde Rieselfähigkeit des Pulvers in der Tablettenpresse, was zum Verkleben und zu schlechtem Tablettieren führt. Probleme, die bei Tabletten aus herkömmlichen Pulvern auftreten, sind z. B. Deckeln, Absplittern und Kleben sowie unbefriedigende Farbe, erhöhte Brüchigkeit und ungenügende Härte.

Die vorliegende Erfindung betrifft neue Vitamin-E-Pulver mit verbesserter Rieselfähigkeit, die direkt zu Tabletten mit verbesserter Härte und geringerer Brüchigkeit verpreßt werden können und 20 bis 60 Gewichtsprozent Vitamin E, 1 bis 25 Gewichtsprozent hydrolysierte Gelatine und etwa 20 bis 30 Gewichtsprozent Natrium- oder Kaliumkaseinat enthalten, mit der Maßgabe, daß das Gewichtsverhältnis von Kaseinat zu Gelatine größer als 1 : 1 ist, wobei sich alle Gewichtsangaben auf das Gesamtgewicht des Pulvers beziehen. Insbesondere betrifft die Erfindung ein direkt zu Tabletten verpreßbares sprühgetrocknetes Vitamin-E-Pulver, das 20 bis 60, vorzugsweise 50 bis 56 Gewichtsprozent Vitamin E, 0 bis 2, vorzugsweise 1 bis 1,5 Gewichtsprozent Fettsäuremonoglycerid, 1 bis 25, vorzugsweise 10 bis 22 Gewichtsprozent hydrolysierte Gelatine, etwa 20 bis etwa 30, vorzugsweise 20 bis 27 Gewichtsprozent Kaseinat und 0,5 bis 2,0, vorzugsweise 1,0 bis 2,0 Gewichtsprozent Siliziumdioxid sowie 0 bis 22, vorzugsweise 9 bis 15 Gewichtsprozent Laktose enthält, wobei sich alle Prozentsätze auf das Gewicht des Pulvers beziehen.

Nach einem weiteren Aspekt der Erfindung wird eine Emulsion hergestellt aus 22 bis 28 Gewichtsprozent Vitamin E, 0 bis 1 Gewichtsprozent Monoglycerid, 0 bis 11 Gewichtsprozent Laktose, 0,4 bis 13 Gewichtsprozent hydrolysierte Gelatine, 8 bis 14 Gewichtsprozent Natriumkaseinat und mindestens 45 Gewichtsprozent, vorzugsweise 45 bis 60 Gewichtsprozent Wasser, jeweils bezogen auf das Gewicht der Emulsion, wobei die Viskosität der Emulsion zwischen 200 und 1000, vorzugsweise zwischen 400 und 600 cP liegt. Die Emulsion wird dann zu einem Vitamin-E-Pulver sprühgetrocknet, und zwar in Gegenwart von 0,5 bis 2,0 Gewichtsprozent Siliziumdioxid, bezogen auf das Gewicht des Pulvers.

Vorzugsweise verwendet man Vitamin-E-Acetat. Die Verwendung von dl-$\alpha$-Tocopherylacetat wird besonders bevorzugt.

Das erfindungsgemäße Pulver enthält etwa 20 bis etwa 30, vorzugsweise 20 bis 27 Gewichtsprozent Kaseinat. Geeignete Kaseinate sind z. B. mit einer geeigneten Base wie Natrium- oder Kaliumhydroxid neutralisiertes Kasein und vorzugsweise Natrium- und Kaliumkaseinat. Kasein, das sich vom Milcheiweiß herleitet, ist ein kolloidales Aggregat aus mehreren Proteinen sowie Phosphor und Kalzium. Bevorzugt verwendet man Natriumkaseinat, bei dem es sich um sprühgetrocknetes, durchreagiertes, farb- und geschmacksreduziertes Milcheiweiß handelt, das aus hochwertigem, eßbarem Kasein hergestellt wurde. Der Natriumkaseinat-Gehalt sollte mehr als etwa 20 Gewichtsprozent, bezogen auf das trockene Pulver, betragen.

Für die Verwendung in dem erfindungsgemäßen Pulver geeignete hydrolysierte Gelatine hat ein Molekulargewicht von etwa 9000 bis etwa 1100 und eine Gelfestigkeit (nach Bloom) von 0. Vorzugsweise verwendet man 1 bis 25 Gewichtsprozent, insbesondere 10 bis 22 Gewichtsprozent. Das Gewichtsverhältnis von Kaseinat zu hydrolysierter Gelatine soll größer als 1 : 1 sein.

Bei der Herstellung der erfindungsgemäßen Pulver wird als sekundäres Emulgiermittel ein Fettsäuremonoglycerid verwendet, und zwar in einer Menge von 0 bis 1,5 Gewichtsprozent, vorzugsweise 1,0 bis 1,5 Gewichtsprozent. Das Monoglycerid ist ein Fettsäure-Glycerinester, in dem das Glycerin nur eine Säuregruppe trägt. Vorzugsweise verwendet man als Monoglycerid 1,0 bis 1,5 Gewichtsprozent einer Mischung aus Glycerinmonostearat und Glycerinmonopalmitat, hergestellt aus hydriertem Talg oder Schweinefett, wobei der Anteil an Monoglycerid etwa 90 Gewichtsprozent und der Anteil an Diglyzeriden etwa 10 Gewichtsprozent beträgt. Das Monoglycerid fördert die Bildung kleiner Emulsionströpfchen, erhöht die Trocknungsgeschwindigkeit und erleichtert das Zerfallen (Auflösen) der Tabletten. Für die Emulsion verwendet man Wasser, vorzugsweise entionisiertes Wasser.

Die erfindungsgemäße Emulsionen werden in Gegenwart von Siliziumdioxid sprühgetrocknet. Vorzugsweise verwendet man Siliziumdioxid mit kolloidaler Teilchengröße, d. h. einer Teilchengröße von 1 nm bis 1 µm. Besonders bevorzugt wird ein Siliziumdioxid, das weniger als 0,2 Gewichtsprozent anderer Substanzen enthält und eine durchschnittliche Primärteilchengröße von etwa 12 nm, eine Oberfläche von etwa 200 m²/g, eine Schüttdichte von etwa 50 g/l, einen Feuchtigkeitsgehalt von höchstens 1,5 Gewichtsprozent (2 Stunden bei 105°C), einen Glühverlust von höchstens 1 Gewichtsprozent (2 Stunden bei 1000°C) und einen pH von 3,6 bis 4,3 (4-gewichtsprozentige wäßrige Disper-

2

sion) aufweist.

Man kann der Emulsion ein Tablettierhilfsmittel in einer Menge von 0 bis 11 Gewichtsprozent, vorzugsweise 4,5 bis 7,5 Gewichtsprozent zusetzen. Geeignete Hilfsmittel dieser Art sind z. B. Disaccharid-Zucker wie Laktose, Saccharose, Maltose und Zellobiose.

Andere mögliche Zusätze sind Konservierungsmittel, wie sie im US-Patent 3 608 083, Spalte 2, Zeilen 27–42, erwähnt werden.

Zur Herstellung der erfindungsgemäßen Emulsionen werden Vitamin E, Wasser, Kaseinat, hydrolysierte Gelatine und gegebenenfalls Monoglycerid, Disacchardid und/oder andere Zusätze in ein Mischgefäß eingetragen. Die durch das Mischen erhaltene Aufschlämmung wird über einen Homogenisator einem Sprühtrockner zugeführt. Die Viskosität der Emulsion ist je nach Wasser- und Kaseinatgehalt verschieden; Sie ist weitgehend unabhängig vom Gehalt an hydrolysierter Gelatine.

Man kann für die Herstellung jedes geeignete Mischgefäß verwenden. Geeignete Homogenisatoren sind solche, die Emulsionströpfchen mit einer Größe von weniger als etwa 2 µm und mit einer Viskosität von bis zu 1000 cP, vorzugsweise zwischen 200 und 1000 cP, erzeugen. Auch läßt sich jeder geeignete Sprühtrockner verwenden, vorzugsweise jedoch vertikal angeordnete Sprühtrockner, die mit einem Zerstäuber ausgerüstet sind, wie z. B. einem rotierenden Versprüher, der mit 14 000 bis 25 000, vorzugsweise 20 000 bis 23 000 Umdrehungen/Minute betrieben wird. Die Eingangstemperatur beträgt 200 bis 216°C und die Austrittstemperatur hängt von Eingangstemperatur und Strömungsgeschwindigkeit ab und liegt gewöhnlich zwischen 88 und 91°C. Bezogen auf das Gewicht des trockenen Pulvers werden 0,5 bis 2,0 Gewichtsprozent Siliziumdioxid in die Trockenkammer eingeführt, vorzugsweise an einer Stelle, an der Unterdruck herrscht. Die Emulsion wird zu einem nicht agglomerierten Pulver sprühgetrocknet.

Bei der Herstellung eines Pulvers, das sich direkt zu pharmazeutischen Vitamintabletten verpressen läßt, ist es wichtig, das Austreten von Öl aus dem Pulver auf ein Minimum zu reduzieren. Ölige Pulver (d. h. Pulver, bei denen ein hoher Anteil des Öls an der Oberfläche austritt) sind ungeeignet, da sie in der Tablettierpresse schlecht fließen oder in der Form der Tablettierpresse kleben bleiben. Solche ungeeigneten Pulver können zu unvollständig ausgebildeten Tabletten führen, und zwar infolge von Kleben im mittleren Bereich der Tablette, Deckeln der Tablette oder Absplittern am Tablettenrand.

Im Gegensatz zu Pulvern, die nach einem herkömmlichen Sprühtrocknungs-Agglomerations-Verfahren hergestellt wurden, sind die Teilchen der erfindungsgemäßen Pulver rieselfähig und nicht agglomeriert. Methoden zur Bestimmung des Schüttwinkels, der ein Maß für die Rieselfähigkeit von Pulvern ist, werden erwähnt in »Some Aspects of the Property of Angle of Repose of Powder« von David Train, J. Pharm. Pharmac., Bd. 10, 1958, S. 127T–135T.

Die Herstellung der Tabletten erfolgt nach herkömmlichen Methoden. Geeignete Tablettierhilfsmittel werden beschrieben in Pharmacentical Technology, Juli 1980, S. 27-35 und 62.

Die erfindungsgemäßen Vitamin-E-Pulver werden zum Beispiel für die direkte Herstellung von Vitamin-E-Tabletten, als Komponente in Multivitamintabletten und als Komponente in Tierfutter-Zusätzen verwendet.

Im folgenden werden die Begriffe Schüttwinkel, Tablettenhärte und Tablettenzerreibbarkeit, wie sie in den Tablettenprüfungen verwendet werden, definiert.

## Tablettenhärte

Als Maß für die Festigkeit von Tabletten (durchschnittlich 10 oder mehr Tabletten) und ihre Beständigkeit gegen Beschädigungen, ausgedrückt in Strong-Cobb-Einheiten (S.C.E.), die nach bekannter Methode mit einem Strong-Cobb-Härteprüfgerät der Strong-Cobb-Arner-Company, Cleveland, Ohio, oder einem gleichwertigen Härteprüfgerät ermittelt wurden, wird der Durchschnitt der erzielten Meßwerte im folgenden als »Härte« angeführt.

## Tablettenfriabilität

Ein Maß für die Neigung von Tabletten (durchschnittlich 10 oder mehr Tabletten) zum Deckeln oder Bröckeln oder zum Abrieb ist im allgemeinen als Gewichtsverlust in Prozent angegeben, der in einer im Journal of the American Pharmacentical Association, Scentific Edition, Bd. 45, S. 114–116 (1956), beschriebenen Prüfung ermittelt wurde. Diese Prüfung wird an 10 oder mehr Tabletten aus jeder Produktionscharge durchgeführt. Zunächst werden die Tabletten entstaubt und gewogen. Danach werden sie 10 Minuten lang der Friabilitätsprüfung in einem »Roche Friabilator« bei 20 Umdrehungen pro Minute unterzogen. Man läßt die Tabletten dabei eine bestimmte Zeit lang rollen und fallen; dann werden sie wieder entstaubt und gewogen. Der Gewichtsverlust gegenüber dem ursprünglichen Gewicht wird in Prozent angegeben. Es ist bekannt, daß eine Wirkstoff enthaltende Tablette, die einen Gewichtsverlust von weniger als etwa 1% (mäßiger Abrieb) aufweist, als zufriedenstellend abriebfest gilt.

**0 062 225**

Schüttwinkel

Um die Rieselfähigkeit der erfindungsgemäßen Pulver aufzuzeigen, wurde ein Schüttwinkel-Test durchgeführt. Methoden zur Bestimmung des Schüttwinkels werden in dem bereits oben angeführten Artikel »Some Aspects of the Property of Angle of Repose of Powder« von David Train beschrieben. Zur Bestimmung des Schüttwinkels von erfindungsgemäßen Pulvern und von Vergleichspulvern wurden jeweils 100 g des zu prüfenden Pulvers in einen 60°-Trichter aus hitzebeständigem Glas (Durchmesser 98 mm, Rohrdurchmesser 15 mm) eingebracht. Der Trichter befand sich in einem Ringständer, wobei der Abstand zwischen Rohrende und Tischplatte 1016 mm betrug. Die Rieselzeit wurde von Beginn bis Ende des Ausströmens in Sekunden und der Schüttwinkel als Winkel zur horizontalen Tischoberfläche gemessen.

In den folgenden Beispielen wird die Erfindung näher erläutert. Alle darin genannten Teile sind, soweit nicht anders angegeben, Gewichtsteile.

Beispiel 1

In einem mit Ankerrührer versehenen Edelstahl-Mantelgefäß wurde eine Aufschlämmung hergestellt. Diese wurde dadurch emulgiert, daß man sie so lange in einem Homogenisator behandelte, bis die Tröpfchengrößen der Emulsion zwischen 1 und 2 µm betrug (Viskosität 400—600 cP).
Die Emulsion enthielt:

| Bestandteil | Teile |
| --- | --- |
| Entionisiertes Wasser | 1565,4 |
| Laktose | 200,8 |
| Natriumkaseinat[1]) | 280,0 |
| Hydrolysierte Gelatine | 138,8 |
| Destilliertes Monoglycerid | 15,7 |
| Vitamin-E-Azetat | 752,9 |

[1]) Laktose und Natriumkaseinat wurden in einem Bandmischer gemischt und erst dann dem entionisierten Wasser bei einer Temperatur zwischen 60 und 65° C zugegeben.

Die Emulsion mit einer Temperatur zwischen 60 und 65° C wurde in einem vertikalen Sprühtrockner (Durchmesser 275 cm) durch eine mit 20 000 bis 23 000 Umdrehungen/Minute rotierende Düse versprüht. Etwa 2 Gewichtsprozent Siliziumdioxid, bezogen auf das Gewicht des Pulvers, werden als Absorptionsmittel an einer Stelle, an der Unterdruck herrscht, in die Trockenkammer eingeführt.
Es wurden 1354 Teile eines sprühgetrockneten Pulvers folgender Zusammensetzung erhalten:

| Bestandteil | Gewichtsprozent, bezogen auf das Gewicht des trockenen Pulvers |
| --- | --- |
| Laktose | 14,32 |
| Natriumkaseinat | 19,98 |
| Hydrolysierte Gelatine | 9,90 |
| Destilliertes Monoglyzerid | 1,12 |
| Vitamin-E-Azetat | 53,69 |
| Siliziumdioxid | 1,0 |

4

**0 062 225**

Der Feuchtigkeitsgehalt des erhaltenen trockenen Pulvers betrug 1,5 Gewichtsprozent, die Teilchengröße des Pulvers zwischen etwa 60 und 100 Mesh (Anzahl der Maschen je Zoll linear). Die durch Gas-Flüssig-Chromatographie ermittelte tatsächliche Vitamin-E-Aktivität belief sich auf 51,8 Gewichtsprozent, der Aschegehalt 1,49 Gewichtsprozent. Die Schüttdichte betrug 46,53 g je 100 ml.

Die Pulver wiesen folgende Eigenschaften auf:

| | |
|---|---|
| Schüttwinkel in Grad | 28—34 |
| Rieselgeschwindigkeit in Sekunden/100 mg | 5,25—5,36 |

Hergestellt wurden Vitamin-E-Kautabletten mit einem Gehalt von etwa 53% Vitamin-E-Pulver, entsprechend 200 Internationalen Einheiten, wobei man eine Tabletten-Standardrezeptur verwendete, die aus direkt verpreßbarem Zucker, Gleitmitteln, einem Schmiermittel, Geschmacksstoffen, Farbstoffen und Zusätzen bestand. Die Herstellung erfolgte in einem Raum, in dem eine Temperatur von 20°C und eine relative Luftfeuchtigkeit von 47% herrschte. Die Dicke der Tabletten wurde so eingestellt, daß man optimale Härte und Friabilität erzielte.

Die Tabletten wurden auf einer Rundläuferpresse bei 30 Umdrehungen/Minute hergestellt. Sie wiesen folgende Eigenschaften auf:

| | |
|---|---|
| Dicke | 6,32—6,43 mm |
| Zerfallszeit | 31—39 Minuten |
| Friabilität | mäßiger Abrieb, kein Absplittern |
| Härte | 9,8 (SCE) |

Vergleichsbeispiel A

Auf entsprechende Weise wurden aus einem handelsüblichen sprühgetrockneten Vitamin-E-Pulver des Standes der Technik Vergleichstabletten hergestellt. Das Pulver enthielt zwischen 40 und 60 Gewichtsprozent hydrolysierte Gelatine, Vitamin-E-Azetat sowie andere herkömmliche Stoffe.

Unter Verwendung der oben beschriebenen Tabletten-Standardrezeptur wurden Vitamon-E-Kautabletten hergestellt und zwar in einem Raum mit einer Temperatur von 21°C und einer relativen Luftfeuchtigkeit von 65%. Unter denselben Tablettierbedingungen wie in Beispiel 1 erhielt man Tabletten mit folgenden Eigenschaften:

| | |
|---|---|
| Dicke | 6,61—6,73 mm |
| Zerfallszeit | 22—27 Minuten |
| Härte | 8,45 SCE |
| Friabilität | mäßiges Absplittern |

Die erfindungsgemäßen Tabletten wiesen höhere Härte und verbesserte Friabilität auf.

Vergleichsbeispiele B—D

Versuche wurden mit erfindungsgemäßen Pulvern und mit Vergleichspulvern durchgeführt. Als Tablettenfüllstoff wird Laktose verwendet. Je mehr sich die Rieselgeschwindigkeit derjenigen von Laktose nähert (Schüttwinkel 30°, Rieselgeschwindigkeit 3,49 Sekunden/100 g), desto besser sind die Rieseleigenschaften des Pulvers. Die Pulver nach der Erfindung wiesen gegenüber denen der Vergleichsbeispiele A und B verbesserte Rieseleigenschaften auf. Die Ergebnisse der Tablettier-Versuche zeigen, daß einige der erfindungsgemäßen Pulver bessere Rieseleigenschaften als die herkömmlichen Pulver haben und daß die Tabletten aus erfindungsgemäßen Pulvern gegenüber denen aus herkömmlichen Pulvern verbesserte Härte und Friabilität aufweisen.

Das in Vergleichsbeispiel B verwendete Pulver enthält 33 bis 35 Gewichtsprozent Gelatine, 2,0 Gewichtsprozent Siliziumdioxid als Absorptionsmittel und 10 Gewichtsprozent Laktose, wobei der Rest Vitamin E-Acetat darstellt.

Das in Vergleichsbeispiel C verwendete Vitamin E-Pulver enthält 56,0 Gewichtsprozent Vitamin E-Acetat, 34,5 Gewichtsprozent Laktose, 7,3 Gewichtsprozent Natriumkaseinat, 1,12 Gewichtsprozent destilliertes Monoglycerid und 1,0 Gewichtsprozent Siliziumdioxid.

Das in Vergleichsbeispiel D verwendete Vitamin-E-Pulver enthält 54,3 Gewichtsprozent Vitamin-E-Azetat, 19,42 Gewichtsprozent Laktose, 25,1 Gewichtsprozent Natriumkaseinat, 1,13 Gewichtsprozent destilliertes Monoglycerid und 0,75 Gewichtsprozent Siliziumdioxid.

5

**Patentansprüche**

1. Vitamin E-Pulver enthaltend 20 bis 60 Gewichtsprozent Vitamin E, 1 bis 25 Gewichtsprozent hydrolysierte Gelatine und etwa 20 bis 30 Gewichtsprozent Natrium- oder Kaliumkaseinat, mit der Maßgabe, daß das Gewichtsverhältnis von Kaseinat zu Gelatine größer als 1 : 1 ist, wobei sich alle Gewichtsangaben auf das Gesamtgewicht des Pulvers beziehen.

2. Vitamin E-Pulver gemäß Anspruch 1, das direkt zu Vitamintabletten verpreßt werden kann und das 50 bis 56 Gewichtsprozent Vitamin E, 0,5 bis 2,0 Gewichtsprozent Siliziumdioxid, 1 bis 25 Gewichtsprozent hydrolysierte Gelatine und etwa 20 bis 30 Gewichtsprozent Natriumkaseinat enthält, mit der Maßgabe, daß das Gewichtsverhältnis von Kaseinat zu Gelatine größer als 1 : 1 ist, wobei sich alle Gewichtsangaben auf das Gesamtgewicht des Pulvers beziehen.

3. Vitamin E-Pulver gemäß Anspruch 1, das direkt zu Vitamintabletten verpreßt werden kann und das im wesentlichen aus 50 bis 56 Gewichtsprozent Vitamin E, 0,5 bis 2,0 Gewichtsprozent Siliziumdioxid, 1 bis 25 Gewichtsprozent hydrolysierte Gelatine, etwa 20 bis etwa 30 Gewichtsprozent Natriumkaseinat und zum Rest auf 100 Gewichtsprozent aus Monoglyzerid und/oder Füllstoff besteht, mit der Maßgabe, daß das Gewichtsverhältnis von Kaseinat zu Gelatine größer als 1 : 1 ist, wobei sich alle Gewichtsangaben auf das Gesamtgewicht des Pulvers beziehen.

4. Pulver nach Anspruch 3, dadurch gekennzeichnet, daß man als Vitamin E Vitamin E-Acetat verwendet.

5. Pulver nach Anspruch 3, dadurch gekennzeichnet, daß man als Fülllmittel Laktose verwendet.

6. Pulver nach Anspruch 3, dadurch gekennzeichnet, daß es 1,0 bis 1,5 Gewichtsprozent Monoglycerid enthält.

7. Pulver nach Anspruch 3, dadurch gekennzeichnet, daß das Siliziumdioxid eine Teilchengröße von 1 nm bis 1 µm hat.

8. Vitamin E-Acetat-Pulver gemäß Anspruch 1, das direkt zu Vitamintabletten verpreßt werden kann und das 50 bis 56 Gewichtsprozent Vitamin-E-Acetat, 1,0 bis 2,0 Gewichtsprozent Siliziumdioxid, 1,0 bis 1,5 Gewichtsprozent destilliertes Monoglycerid, 0 bis 22 Gewichtsprozent Laktose, 10 bis 22 Gewichtsprozent hydrolysierte Gelatine und 20 bis 27 Gewichtsprozent Natriumkaseinat enthält, mit der Maßgabe, daß das Gewichtsverhältnis von Kaseinat zu Gelatine größer als 1 : 1 ist, wobei sich alle Gewichtsangaben auf das Gesamtgewicht des Pulvers beziehen.

9. Pulver nach Anspruch 8, enthaltend 9 bis 15 Gewichtsprozent Laktose.

10. Verfahren zur Herstellung von sprühgetrocknetem Vitamin E-Pulver gemäß Anspruch 1, dadurch gekennzeichnet, daß man

A) 45 bis 60 Gewichtsprozent entionisiertes Wasser, 22 bis 28 Gewichtsprozent Vitamin E-Acetat, 8 bis 14 Gewichtsprozent Natriumkaseinat, 0,4 bis 13 Gewichtsprozent hydrolysierte Gelatine, 0 bis 1 Gewichtsprozent Monoglycerid und 0 bis 11 Gewichtsprozent Laktose zu einer Emulsion mit einer Teilchengröße von weniger als etwa 2 µm und einer Viskosität von bis zu 1000 cP verarbeitet, mit der Maßgabe, daß das Gewichtsverhältnis von Kaseinat zu Gelatine größer als 1 : 1 ist, und

B) die Emulsion in Gegenwart von 0,5 bis 2,0 Gewichtsprozent Siliziumdioxid zu einem nicht agglomerierten Pulver sprühtrocknet.

11. Direkt gepreßte Vitamin-E-Acetat-Tabletten, hergestellt aus einem Pulver gemäß Anspruch 1, enthaltend Vitamin-E-Acetat, hydrolysierte Gelatine und Natriumkaseinat.

12. Tabletten nach Anspruch 10, dadurch gekennzeichnet, daß sie eine durchschnittliche Härte von 8,7 bis 11,7 (in Strong-Cobb-Einheiten) und leichte bis mäßige Friabilität aufweisen.

**Claims**

1. A vitamin E powder containing 20 to 60 percent by weight of vitamin E, 1 to 25 percent by weight of a hydrolyzed gelatin and about 20 to 30 percent by weight of sodium or potassium caseinate, with the proviso that the weight ration of caseinate to gelatin is higher than 1 : 1, all percentages by weight being based on the total weight of the powder.

2. A vitamin E powder as claimed in claim 1, which can be directly formed by compression into vitamin tablets and which contains 50 to 56 percent by weight of vitamin E, 0.5 to 2.0 percent by weight of silicon dioxide, 1 to 25 percent by weight of a hydrolyzed gelatin, and about 20 to 30 percent by weight of sodium caseinate, with the proviso that the weight ratio of caseinate to gelatin is higher than 1 : 1, all percentages by weight being based on the total weight of the powder.

3. A vitamin E powder as claimed in claim 1, which can be directly formed by compression into vitamin tablets and consists essentially of 50 to 56 percent by weight of vitamin E, 0.5 to 2.0 percent by weight of silicon dioxide, 1 to 25 percent by weight of a hydrolyzed gelatin, about 20 to about 30 percent by weight of sodium caseinate, and of a monoglyceride and/or a filler as the remainder giving 100 percent by weight, with the proviso that the weight ratio of caseinate to gelatin is higher than 1 : 1, all percentages by weight being based on the total weight of the powder.

4. A powder as claimed in claim 3, wherein vitamin E acetate is used as vitamin E.

5. A powder as claimed in claim 3, wherein lactose is used as filler.

6. A powder as claimed in claim 3, containing from 1.0 to 1.5 percent by weight of monoglyceride.

7. A powder as claimed in claim 3, wherein the silicon dioxide has a particle size of 1 nm to 1 μm.

8. A vitamin E acetate powder as claimed in claim 1, which can be directly formed by compression into vitamin tablets and contains 50 to 56 percent by weight of vitamin E acetate, 1.0 to 2.0 percent by weight of silicon dioxide, 1.0 to 1.5 percent by weight of distilled monoglyceride, 0 to 22 percent by weight of sodium caseinate, with the proviso that the weight ration of caseinate to gelatin is higher than 1 : 1, all percentages by weight being based on the total weight of the powder.

9. A powder as claimed in claim 8, containing 9 to 15 percent by weight of lactose.

10. A process for the preparation of a spray-dried vitamin E powder as claimed in claim 1, wherein

(A) 45 to 60 percent by weight of deionized water, 22 to 28 percent by weight of vitamin E acetate, 8 to 14 percent by weight of sodium caseinate, 0.4 to 13 percent by weight of a hydrolyzed gelatin, 0 to 1 percent by weight of monoglyceride, and 0 to 11 percent by weight of lactose, with the proviso that the weight ratio of caseinate to gelatin is higher than 1 : 1, are processed into an emulsion having a particle size of less than about 2 μm and a viscosity of up to 1000 cps, and

(B) the emulsion is spray-dried in the presence of from 0.5 to 2.0 percent by weight of silicon dioxide to form a non-agglomerated powder.

11. Vitamin E acetate tablets directly formed by compression and prepared from a powder as claimed in claim 1, containing vitamin E acetate, a hydrolyzed gelatin and sodium caseinate.

12. Tablets as claimed in claim 10, having an average hardness of from 8.7 to 11.7 (in Strong Cobb units) and slight to moderate friability.

## Revendications

1. Vitamine E-poudre, contenant 20 à 60% en poids de vitamine E, 1 à 25% en poids de gélatine hydrolysée et environ 20 à 30% en poids de caséinate de sodium ou de potassium, sous réserve que le rapport pondéral du caseinate à la gélatine soit supérieur à 1/1, toute les indications pondérales se rapportant au poids total de la poudre.

2. Vitamine E-poudre selon la revendication 1, qui peut être mise à la presse directement en tablettes de vitamines, et qui contient 50 à 56% en poids de vitamine E, 0,5 à 2,0% en poids de bioxyde de silicium, 1 à 25% en poids de gélatine hydrolysée et environ 20 à 30% en poids de caseinate de sodium, sous réserve que le rapport pondéral du caseinate à la gélatine soit supérieur à 1/1, toutes les indications pondérales se rapportant au poids total de la poudre.

3. Vitamine E-poudre selon la revendication 1, qui peut être mise à la presse directement en tablettes de vitamines, et qui est constituée essentiellement de 50 à 56% en poids de vitamine E, 0,5 à 2,0% en poids de bioxyde de silicium, 1 à 25% en poids de gélatine hydrolysée et environ 20 à 30% en poids de caseinate de sodium et, pour le reste, de 100% en poids de monoglycéride et/ou de charge, sous réserve que le rapport pondéral du caseinate à la gélatine soit supérieur à 1/1, toutes les indications pondérales se rapportant au poids total de la poudre.

4. Poudre selon la revendication 3, caractérisé par le fait que l'on utilise, comme vitamine E, de la vitamineacétate.

5. Poudre selon la revendication 3, caractérisé par le fait que l'on utilise, comme charge, du lactose.

6. Poudre selon la revendication 3, caractérisé par le fait qu'elle contient 1,0 à 1,5% en poids de monoglycéride.

7. Poudre selon la revendication 3, caractérisé par le fait que le bioxyde de silicium a une grosseur de particules de 1 nm à 1 μm.

8. Vitamine E-acétate-poudre selon la revendication 1, qui peut être mise à la presse directement en tablettes de vitamine et qui contient 50 à 56% en poids de vitamine E-acétate, 1,0 à 2,0% en poids de bioxyde de silicium, 1,0 à 1,5% en poids de monoglycéride distillé, 0 à 22% en poids de lactose, 10 à 22% en poids de gélatine hydrolysée et 20 à 27% en poids de caseinate de sodium sous réserve que le rapport pondéral du caseinate à la gélatine soit supérieur à 1/1, toutes les indications pondérales se rapportant au poids total de la poudre.

9. Poudre selon la revendication 8, contenant 9 à 15% en poids de lactose.

10. Procédé de préparation de vitamine E-poudre selon la revendication 1, séchée par pulvérisation, caractérisé par le fait que

A) on met en émulsion 45 à 60% en poids d'eau déionisée, 22 à 28% en poids de vitamine E-acétate, 8 à 14% en poids de caseinate de sodium, 0,4 à 13% en poids de gélatine hydrolysée, 0 à 1% en poids de monoglycéride et 0 à 11% en poids de lactose, l'émulsion ayant une grosseur de particules inférieure à environ 2 μm et une viscosité allant jusqu'à 1000 cP, sous réserve que le rapport pondéral du caseinate à la gélatine soit supérieur à 1/1 et

B) on sèche par pulvérisation l'émulsion en présence de 0,5 à 2,0% en poids de bioxyde de silicium, pour obtenir une poudre non agglomérée.

11. Tablettes de vitamine E-acétate directement pressées et préparées à partir d'une poudre selon la revendication 1, contenant de la vitamine E-acétate, de la gélatine hydrolysée et du caseinate de sodium.

12. Tablettes selon la revendication 10, caractérisées par le fait qu'elles possèdent une dureté moyenne de 8,7 à 11,7 (en unités Strong-Cobb) et une friabilité légère à modérée.